# EUROPEAN PATENT SPECIFICATION

(11) **EP 2 014 306 B1**
(45) Date of publication and mention of the grant of the patent: **25.02.2015**
(21) Application number: 08009119.2
(22) Date of filing: 16.05.2008
(51) Int. Cl.: A61K 47/48

(54) **Metal-polysaccharide conjugates: compositions and synthesis**
Metall-Polysaccharid-Konjugate: Zusammensetzungen und Synthese
Conjugués polysaccharide-métal: compositions et synthèse

(30) Priority: 04.06.2007 US 933034 P
(43) Date of publication of application: 14.01.2009
(73) Proprietor: Taiwan Hopax Chems. Mfg. Co., Ltd., Kaohsiung 813 (TW)
(72) Inventor: Wei, I-Chien, Daliao Township Kaohsiung County 831 (TW); Yang, David J., Sugar Land, TX 77478 (US); Yu, Dong-Fang, Houston, TX 77030 (US)
(74) Representative: UEXKÜLL & STOLBERG

(56) References cited:
- EP-A- 1 698 351
- EP-A2- 0 280 474
- ISHII H. ET AL.: "Synthesis of chitosan-amino acid conjugates and their use in heavy metal uptake" INTERNATIONAL JOURNAL OF BIOLOGICAL MACROMOLECULES, vol. 17, no. 1, 1995, pages 21-23, XP002486318
- MAEDA M ET AL: "ANTITUMOR ACTIVITY OF A NEW SERIES OF PLATINUM COMPLEXES: TRANS(+-)-1,2-CYCLOHEXANEDIAMMINEPLATINUM( II) CONJUGATED TO ACID POLYSACCHARIDES" ANTI-CANCER DRUGS, RAPID COMMUNICATIONS, OXFORD, vol. 4, no. 2, 1 April 1993 (1993-04-01), pages 167-171, XP008068856 ISSN: 0959-4973
- ZHANG JING-SHI ET AL.: "Effects of a cisplatin-chondroitin sulfate A complex in reducing the nephrotoxicity of cisplatin" ARCHIVES OF TOXICOLOGY, vol. 74, no. 6, August 2000 (2000-08), pages 300-307, XP002486319
- ZHANG JING-SHI ET AL.: "Stability of a cisplatin-chondroitin sulfate A complex in plasma and kidney in terms of protein binding" BIOLOGICAL AND PHARMACEUTICAL BULLETIN, vol. 24, no. 8, August 2001 (2001-08), pages 970-972, XP002486320
- ZHANG JING-SHI ET AL.: "Molecular-weight-dependent pharmacokinetics and cytotoxic properties of cisplatin complexes prepared with chondroitin sulfate A and C" INTERNATIONAL JOURNAL OF PHARMACEUTICS (KIDLINGTON), vol. 240, no. 1-2, 20 June 2002 (2002-06-20), pages 23-31, XP002486321
- MATEO CESAR ET AL: "Stabilization of multimeric enzymes via immobilization and further cross-linking with aldehyde-dextran", METHODS IN BIOTECHNOLOGY HUMANA PRESS INC, 999 RIVERVIEW DR, STE 208, TOTOWA, NJ 07512-1165 USA SERIES : METHODS IN BIOTECHNOLOGY, 2006, pages 129-141, ISSN: null

## Description

### TECHNICAL FIELD

Embodiments of the present invention relate to conjugates of metal and polysaccharides via monomeric amino acids according to the claims. These polysaccharide conjugates may be used to induce cancer cell death and in cancer therapy.

### TECHNICAL BACKGROUND

Angiogenesis processes are involved in the tumor vasculature density and permeability. An increased understanding of these processes as well as cell cycle regulation and cell signaling agents has opened a new era in the treatment of various tumors. Despite the outstanding advances made in the field of angiogenesis, some significant limitations still remain in the treatment of cancer, tumors and other diseases having an angiogenic component via drug agents. One of the most significant limitations at this time relates to the delivery of cytotoxic drugs instead of cytostatic drugs *in vivo.*

The effectiveness of platinum conjugates against tumor activity has been demonstrated. For instance, cisplatin, a widely used anticancer drug, has been used as alone or in combination with other agents to treat breast and ovarian cancers. Cisplatin, also known as cis-diamminedichloroplatinum (II) (CDDP), is a simple molecule with Pt conjugated to NH₃ molecules. Cisplatin causes cell arrest at S-phase and that leads to mitotic arrest of proliferating cells. Cisplatin also decreases expression of vascular endothelial growth factor (VEGF) during chemotherapy, thus limiting angiogenesis. Cisplatin is effective in the treatment of majority solid tumors. However, clinical applications using cisplatin are limited due to significant nephrotoxicity, myelosuppression, drug resistance, gastrointestinal toxicity, neurotoxicity and other side effects (e.g. vomiting, granulocytopenia and body weight loss). In addition, cisplatin is formulated in bulky vehicles with poor water solubility, which impairs its therapeutic efficacy. Chemical modifications of various platinum conjugates have been made to increase its hydrophilicity, reduce its side effects and improve its therapeutic efficacy, however, these conjugates still present serious drawbacks.

Maeda M. et al. (1993), Anti-Cancer Drugs, volume 4, pp. 167-171, discloses a conjugate of chondroitin sulfate to a platinum drug with antitumor activity.

### SUMMARY

The current invention, in one embodiment, includes a polysaccharide conjugate according to the claims. This conjugate has a polysaccharide and at least one monomeric amino acid having an O-group covalently bound to the polysaccharide. The conjugate also has at least one metal conjugated by the O-group of the amino acid.

According to another embodiment, the invention provides a method of synthesizing a polysaccharide conjugate according to the claims by covalently bonding a monomeric amino acid having an O-group to a polysacchride and by conjugating a metal to the O-group to form a polysaccharide conjugate.

According to a third embodiment, the invention relates to a polysaccharide conjugate according to the claims for use in a method of killing a cancer cell by administering to the cell an effective amount of said polysaccharide conjugate. This conjugate has a polysaccharide and at least one monomeric amino acid having an O-group covalently bound to the polysaccharide. The conjugate also has at least one metal conjugated by the O-group of the amino acid.

### BRIEF DESCRIPTION OF THE DRAWINGS

The following figures form part of the present specification and are included to further demonstrate certain aspects of the present invention. The invention may be better understood by reference to one or more of these drawings in combination with the description of embodiments presented herein.
Figure 1 illustrates three types of metal-polysaccharide conjugates according to embodiments of the present invention. "AA" designates an amino acid. "M" designates a metal. In Figure 1A, only one or a few amino acid groups and conjugated metal are present. In Figure 1B, an intermediate number of amino acid groups and conjugated metal are present. In Figure 1C the maximum or nearly the maximum possible amino acid groups and conjugated metal are present.
Figure 2 shows one method (Method A) of synthesis of a platinum analogue (II) and (IV) -polysaccharide conjugate, according to an embodiment of the present invention.
Figure 3 shows another method (Method B) of synthesis of a polysaccharide-platinum analogue (II) and (IV) conjugate, according to an embodiment of the present invention.
Figure 4 shows the effect of a platinum-polysaccharide conjugate, according to an embodiment of the present invention, on inhibition of platinum-resistant ovarian cancer cells (2008 c13) at 48 hours (A) and 72 hours(B).
Figure 5 shows the effect of a platinum-polysaccharide conjugate, according to an embodiment of the present invention, on inhibition of platinum-sensitive ovarian cancer cells (2008) at 48 h (A) and 72h (B).
Figure 6 shows the results of flow cytometry showing the apoptotic effects of cisplatin (CDDP) (A) and a platinum-polysacchardide conjugate (PC), according to an embodiment of the present invention, (B) on a platinum-resistant ovarian cancer cell line, 2008-c13, at 48 hours.
Figure 7 shows the percentages of apoptotic cells detected by flow cytometry in the platinum-resistant ovarian cancer cell line 2008-c13 treated with a platinum-polysaccharide conjugate (PC), according to an embodiment of the present invention, or cisplatin (CDDP) at various concentrations for 48 hours (A) and 72 hours (B).
Figure 8 shows the percentage of apoptotic cells detected by TUNEL assay in the platinum-resistant ovarian cancer cell line 2008-c13 treated with a platinum-polysaccharide conjugate (PC), according to an embodiment of the present invention, or cisplatin (CDDP) at various concentrations for 48 hours.

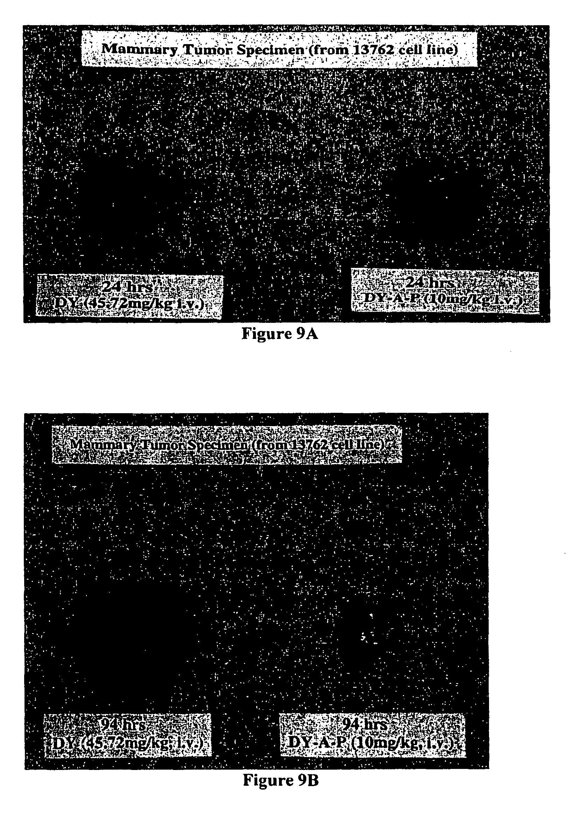

Figure 9 shows the *in vivo* effects of a platinum-polysaccharide conjugate, according to an embodiment of the present invention, against breast tumor growth at 24 hours (A) and 94 hrs (B) (single dose, Pt 10mg/kg). The tumors designated DY were taken from an animal administered only chondriotin. the tumors designated DP-A-P were taken from an animal administered the platinum-polysaccharide conjugate. In Figure 9A, the tumor on the left measured (2.08 cm x 2.58 cm x 1.96 cm)/2 for a volume of 5.2591 cm³. The tumor on the right measured (2.20 cm x 2.37 cm x 2.02 cm)/2 for a volume of 5.2661 cm³. In Figure 9B, the tumor on the left measured (2.99 cm x 3.29 cm x 2.92 cm)/2 for a volume of 14.3622 cm³. The tumor on the right measured (1.11 cm x 1.84 cm x 0.86 cm)/2 for a volume of 0.8782 cm³.
Figure 10 shows H & E staining of tumors to show necrosis at 24 and 94 hrs post-administration of a platinum-polysaccharide conjugate, according to an embodiment of the present invention, or chondroitin alone. Figure 10A shows a mammary tumor (13762) at 24 hrs administered chondroitin. Figure 10B shows a mammary tumor (13762) at 24 hrs administered a platinum-polysaccharide conjugate. Figure 10C shows a mammary tumor (13762) at 94 hrs administered chondroitin. Figure 10D shows a mammary tumor (13762) at 94 hrs administered a platinum-polysaccharide conjugate.
Figure 11 shows a Western blot of PARP protein from 2008-c13 cells treated with either platinum-polysaccharide conjugate (PC) or cisplatin (CDDP).
Figure 12 shows the results of flow cytometric analysis of the cell cycle of 2008-c13 cells platinum-polysaccharide conjugate (PC) or cisplatin (CDDP) after 48 hours.
Figure 13 shows a Northern blot for p21 transcript (Figure 13A) and a Western blot for expressed p21 (Figure 13B) in 2008-c13 cells treated with low doses of platinum-polysaccharide conjugate (PC) or cisplatin (CDDP).
Figure 14A shows Flow cytometric analysis of the dose-dependent increase of the sub-G₁ fraction after 48 hours-exposure to cisplatin (CDDP) or conjugate (PC or DDAP). At the same doses, PC induced substantially more sub-G₁ cells than did CDDP in platinum-resistant 2008.C13 cells. Figure 14B shows the percentage of the sub-G₁ fraction in 2008.C13 cells after 48 hours-exposure to CDDP or PC (DDAP).
Figure 15 shows a TUNEL assay of apoptosis induced by *cis*-diamminedichloroplatinum(II) (CDDP) and diammine dicarboxylic acid platinum (PC or DDAP) after 48 hours of drug exposure. In Figure 15A, the apoptotic morphology is indicated by brown particles. In Figure 15B, the percentage of cells with apoptotic morphology. Columns, mean of three independent experiments; bars, SE.
Figure 16 shows a Western blot analysis of cleaved caspase-3 and specific poly (ADP-ribose) polymerase (PARP) cleavage in 2008.C13 cells treated with *cis*-diamminedichloroplatinum(II) (CDDP) or diammine dicarboxylic acid platinum (PCor DDAP). GAPDH, glyceraldehyde-3-phosphate dehydrogenase.
Figure 17A shows the cell-cycle distribution after treatment with *cis*-diamminedichloroplatinum(II) (CDDP) or diammine dicarboxylic acid platinum (PC or DDAP) for 48 hours in the 2008.C13 cell line. G₁, G₂, M, and S indicate cell phases. Figure 17B shows a Western blot analysis of p21 and cyclin A expression in 2008.C13 cells after exposure to *cis*-diamminedichloroplatinum(II) (CDDP) or diammine dicarboxylic acid platinum (PC or DDAP) for 48 hours. GAPDH, glyceraldehyde-3-phosphate dehydrogenase.

### DETAILED DESCRIPTION

The present invention, in certain embodiments, includes metal-polysaccharides conjugates according to the claims, methods for their synthesis, and uses thereof, including inducing cancer cell death and treatment of cancer. In particular, the conjugate may include a polysaccharide with at least one monomeric amino acid attached. This amino acid may then conjugate the metal. In many embodiments, there may be multiple monomeric amino acids attached, which allows for conjugation of multiple metal groups. The conjugates may be of any size, but in certain embodiments, the conjugate may be designed so that each molecule is at least 10,000 daltons, for example between 10,000 and 50,000 daltons, to limit excretion through the kidneys. In a particular embodiment, the polysaccharide conjugate may have a molecular weight of between about 20,000 daltons to about 50,000 daltons, more particularly it may be between about 26,000 to about 30,000 daltons.

The polysaccharide is chondroitin. In one particular embodiment, it may be chondroitin A. Although the present invention is not limited to a particular mode of action, such polysaccharides may facilitate uptake through the vasculature and delivery to cancer cells. This may be particularly true in areas undergoing angiogenesis, such as most tumors. The end product molecular weight range of 20,000-50,000 daltons will help achieve vascular-based therapy.

The amino acid is covalently bonded to the polysaccharide. The amino acid is in monomeric form, such that individual monomers are attached separately to the polysaccharide. The amino acid is aspartic acid. Due to bond distance between two carboxylic acid and better tumor uptake specificity, aspartic acid is preferred. The amino acids may be in L-form, or D-form, or a racemic mixture of L- and D-forms. Amino acid in L-form is preferred for optimal tumor uptake. Aspartic acid has been selected because a single aspartic acid monomer is able to conjugate a metal on its own. Additionally, aspartic acid is not produced by mammalian cells, but is a necessary nutrient, making it likely to be taken up by rapidly growing tumor cells.

The amino acid may comprise between about 10% to about 50%, by weight of the polysaccharide conjugate.

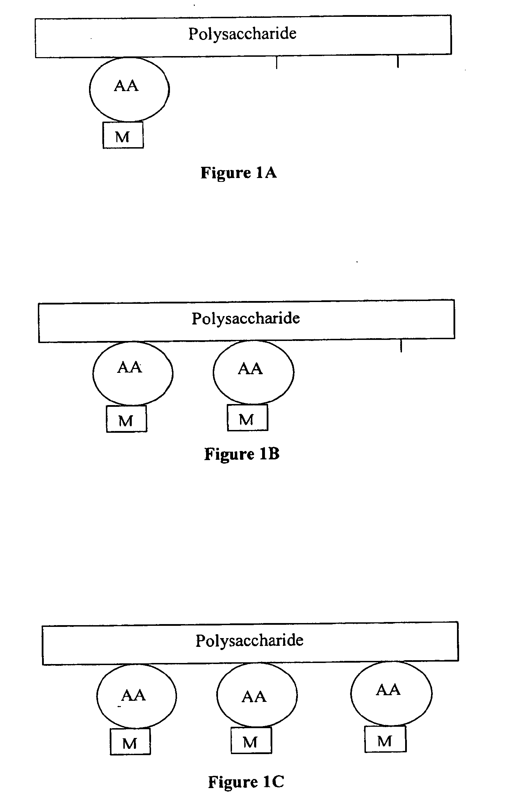

The degree of saturation of amino acid attachment points on the polysaccharide may vary. For example, as shown in Figure 1A, only one amino acid may be attached. Very low degrees of saturation, such as 5% or less, 10% or less, or 20% or less may also be achieved. Figure 1B illustrates a conjugate with an intermediate degree of saturation, such as approximately 30%, approximately 40%, approximately 50%, or approximately 70%. Figure 1C illustrates a conjugate with very high degrees of saturation, such as 80% or greater, 90% or greater, 95% or greater, or substantially complete saturation. Although in Figure 1 each amino acid has a conjugated metal, in many actual examples, there will be degrees of saturation of the available amino acids by the metal, such as less than 5%, 10% or 20%, approximately 30%, approximately 40%, approximately 50%, or approximately 70%, greater than 80%, 90%, 95%, or substantially complete saturation.

The metal is platinum and is part of a larger molecule, such as a drug. The metal is conjugated to the polysaccharide-amino acid backbone via O-groups of the amino acid monomers.

In one embodiment, the metal may be between 15 percent to about 30 percent, by weight of the polysaccharide conjugate.

In one example embodiment, the conjugate includes chondroitin A covalently bonded to aspartic acid monomers, which conjugate platinum in a platinum-containing compound. In one variation the platinum may be platinum (II) and in another variation the platinum may be platinum (IV).

The conjugate may be water soluble. For example, it may have a solubility of at least approximately 20 mg (metal equivalent)/ml water. The conjugate may be provided in a variety of forms, such as an aqueous solution or a powder. The conjugate and its formulations may be sterilized. For example, it may be provided as a sterilized powder.

The conjugate may be synthesized, according to one embodiment of the invention, by separately covalently bonding one or more amino acid monomers to a polysaccharide. Then a metal may be provided for conjugation by the amino acid monomers. According to another embodiment of the invention, the metal may be conjugated to the amino acid monomers, then one or more of the amino acid monomers may be covalently bonded to the polysaccharide.

Conjugates of the present invention may be used to kill cancer or tumor cells and thus may treat cancer or tumors. Conjugates may target tumors, particularly solid tumors. This may be verified, for example, through radiolabeled variations of the compounds, such as a polysaccharide-amino acid backbone conjugated to ^{99m}Tc, which allows gamma imaging. Cytotoxic agents with a metal component may be conjugated to the polysaccharide-amino acid backbone to reduce their cytotoxic effects. For example, the cytotoxic agents maybe released gradually from the polyssaccharide, decreasing acute systemic toxicity. Additionally, the therapeutic index (toxicity/efficacy) of drugs with poor water solubility or tumor targeting capacity may be increased by conjugating those drugs to the polysaccharide-polymer backbone.

In specific example embodiments, platinum-containing conjugates may be able to inhibit cancer cell growth at lower doses than cisplatin. Further, platinum-containing conjugates may also be able to inhibit cell growth of cisplatin-resistant cancer cells, particularly ovarian cancer cells.

Any type of cancer or tumor cell may be killed or have its growth inhibited by selected conjugates of the present invention. However, solid tumors may respond best to these conjugates. Example cancers that may be susceptible to certain conjugates of the present invention include: ovarian cancer, cisplatin-resistant ovarian cancer, pancreatic cancer, breast cancer, sarcoma, uterine cancer, and lymphoma.

In addition to cancer, certain conjugates of the present invention may be able to target and inhibit cells involved in the development and progression of the following diseases: HIV, autoimmune diseases (e.g. encephalomyelitis, vitiligo, scleroderma, thyroiditis, and perforating collagenosis), genetic diseases (e.g xeroderma pigmentosum and glucose-6-phosphate dehydrogenase deficiency), metabolic diseases (e.g. diabetes mellitus), cardiovascular diseases, neuro/psychiatric diseases and other medical conditions (e.g. hypoglycemia and hepatic cirrhosis).

### EXAMPLES

The following examples are included to demonstrate specific embodiments of the invention. It should be appreciated by those of skill in the art that the techniques disclosed in the examples that follow represent techniques discovered by the inventors to function well in the practice of the invention.

### Example I - Synthesis of platinum analogue (II) and (IV)-polysaccharide conjugate

### Method A.

Cis-1,2-Diaminocyclohexane sulfatoplatinum (II) (cis-1,2-DACH-Pt-SO₄) was synthesized via a two-step procedure. In the first step, cis-1,2-DACH-PtI₂ complex was synthesized by mixing a filtered solution of K₂PtCl₄(5.00g, 12 mmol) in 120 ml of deionized water with KI (20.00g in 12 ml of water, 120 mmol) and was allowed to stir for 5 min. To this solution one equivalent of the cis-1,2-DACH(1.37g, 1.487 ml, 12 mmol) was added. The reaction mixture was stirred for 30 min at room temperature. The obtained yellow solid was separated by filtration and then washed with a small amount of deionized water. The final product was dried under vacuum, which yielded cis-1,2-DACH-PtI₂ (6.48g, 96%). In the second step, cis-1,2-DACH-PtI₂ (without further purification from step 1, 6.48g, 11.5 mmol) was added as a solid to an aqueous solution of Ag₂SO₄ (3.45g, 11 mmol). The reaction mixture was left stirring overnight at room temperature. The AgI was removed by filtration and the filtrate was freeze dried under vacuum, which yielded yellow cis-1,2-DACH-Pt(II)SO₄ (4.83g, 99%). Elemental analysis showed Pt: 44.6% (w/w).

To a stirred solution of chondroitin (1g, MW. 30,000-35,000) in water (5 ml), sulfo-NHS (241.6 mg, 1.12 mmol) and 3-ethylcarbodiimidel-ethyl-3-(3-dimethylaminopropyl) carbodiimide-HCl (EDC) (218.8 mg, 1.15 mmol) (Pierce Chemical Company, Rockford, IL) were added. L-aspartic acid sodium salt (356.8 mg, 1.65 mmol) was then added. The mixture was stirred at room temperature for 24 hours. The mixture was dialyzed for 48 hours using a Spectra/POR molecular porous membrane with cut-off at 10,000 (Spectrum Medical Industries Inc., Houston, TX). After dialysis, the product was filtered and freeze dried using lyophilizer (Labconco, Kansas City, MO). The product, aspartate-chondroitin (polysaccharide), in the salt form, weighed 1.29 g. A similar technique was used to prepare condroitin having glutamic acid and alanine, glutamic acid and asparagine, glutamic acid and glutamine, glutamic acid and glycine, and glutamic acid and one aspartic acid conjugated with alanine, asparagine, glutamine, and glycine.

Cis-1,2-DACH-Pt (II) SO₄ (500mg, 1.18 mmol) was dissolved in 10 ml of deionized water, and a solution of aspartate-chondroitin (1.00g in 15 ml of deionized water) was added. The solution was left stirring for 24 hr at room temperature. After dialysis (MW:10,000) and lyophilization, the yield of cis-1,2-DACH-Pt (II) -polysaccharide was 1.1462g.

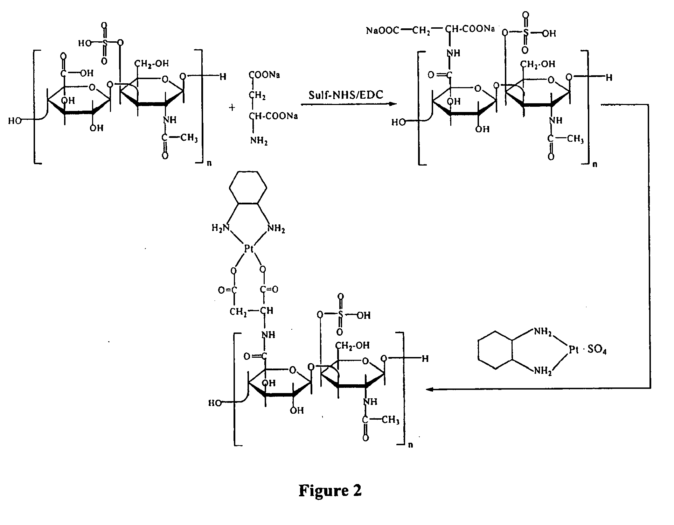

The platinum-polysaccharide conjugate, Cis-1,2-DACH-dichloro-Pt (IV)-aspartate-chondroitin (PC) was synthesized as follows: the above solution was added dropwise 2.5 ml of 30% aqueous hydrogen peroxide. After 24 hr, HCl (75ml of 0.02 N) was added and left stirring for 24 hr at room temperature, dialyzed (MW:10,000) by deionized water for overnight and freeze dried under vacuum. The final product obtained was 1.15g. Elemental analysis showed Pt: 21.87% (w/w). The synthetic scheme is shown in Figure 2.

### Method B.

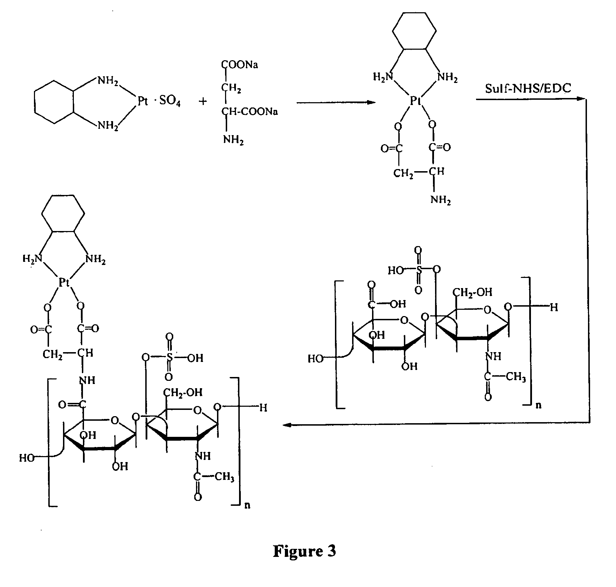

The Cis-1,2-DACH-Pt (II) SO₄ or Cis-1,2-DACH-dichloro-Pt (IV) (500mg, 1.18 mmol) was dissolved in 10 ml of deionized water, and a solution of aspartic acid (67 mg, 0.5 mmol) in 2 ml of deionized water was added. The solution was left stirring for 24 hr at room temperature. After dialysis and lyophilization, the cis-1,2-DACH-Pt -aspartate was reacted with chondroitin (1g, MW. 30,000-35,000) in water (5 ml), sulfo-NHS (241.6 mg, 1.12 mmol) and 3-ethylcarbodiimidel-ethyl-3-(3-dimethylaminopropyl) carbodiimide-HCl (EDC) (218.8 mg, 1.15 mmol) (Pierce Chemical Company, Rockford, IL). The synthetic scheme is shown in Figure 3.

### Example 2 - In vitro cell culture assay:

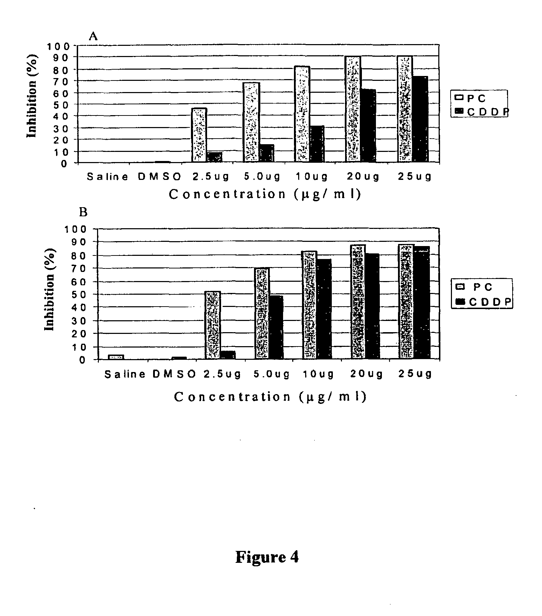

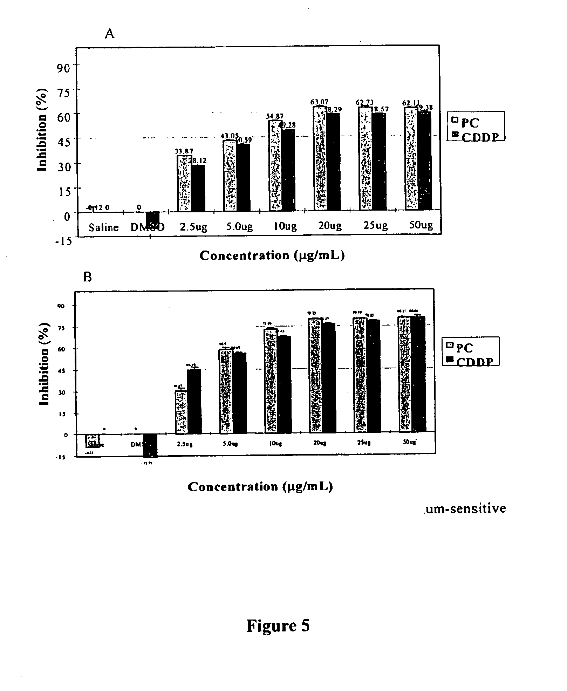

To evaluate cytotoxicity of cisplatin and platinum (II)-polysaccharide conjugate (PC) prepared as described above using Method A against mammary tumor cells, two human tumor cell lines were selected: the 2008 line and its platinum-resistant subline, 2008-c13. All cells were cultured at 37°C in a humidified atmosphere containing 5% CO₂ in RPMI 1640 medium supplemented with 10% fetal bovine serum and glutamine (2 mM). 2008 or 2008.C 13 cells were seeded into 96-well plates (4,000 cells/well) and maintained in RPMI 1640 medium for 24 hours. Next, cells were treated with PC or CDDP at concentrations of 2.5, 5, 10, 20, 25, and 50 µg/mL for 48 and 72 hours. Controls were treated with DMSO or PBS. After cells were treated, their growth and viability were determined by incubating the cells for 1 to 2 hours at 37°C with 20 µL of tetrazolium substrate. Absorbence was measured at 450 nm using a 96-well Synergy HT-microplate reader (Biotek, Winooski, Vermont). The rate of cell growth inhibition was expressed as a percentage as follows: %= 100-(OD_{controls-} OD_{treated cells})/ OD_{controls}. The experiments were repeated separately three times. Methylene tetrazolium (MTT) dye assay determined the amount of viable cells. Cellular protein content was determined by Lowry assay. The drug concentration that inhibits 50% of cell growth (IC-50) was then determined. Data are expressed as the percentage differences compared with controls (OD of cells after treatment/OD of cells without treatment). An illustrated cell inhibition curves are shown in Figures 4 and 5.

The findings showed that the sensitivity of cells to exposure to the IC-50 of platinum-polysaccharide conjugate (PC) was 5.7 times greater than that to the exposure to the IC-50 of cisplatin (CDDP) (Figure 4) in the platinum-resistant ovarian cancer cell line but not in the platinum-sensitive ovarian cancer line (2008) (Figure 5). In particular, concentrations of platinum-polysaccharide conjugate (PC) at 2.5 and 5 µg/mL enhanced tumor killing by 5.9 and 4.6 times, respectively, at 48 hours compared with cisplatin (CDDP) (Figure 4A) and by 9.3 and 1.5 times, respectively, at 72 hours compared with cisplatin (CDDP) (Figure 4B). The data indicated that low doses of platinum-polysaccharide conjugate (PC) significantly inhibit cell growth of platinum-resistant ovarian cancer cells.

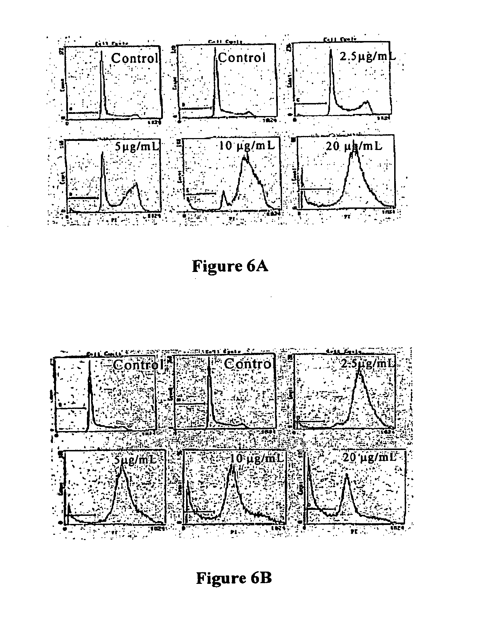

To determine the effectiveness of platinum-polysaccharide conjugate (PC) against platinum-resistant ovarian cancer cells, 2008-c13 cells (0.5 x 10⁻⁶) were treated with platinum-polysaccharide conjugate (PC). The cells were trypsinized and centrifuged at 2500 rpm for 5 min. After being washed with 1x PBS two times, cells were fixed with 70% ethanol overnight, washed twice with 1x PBS, and resuspended in 1 mL of propidium iodide (PI) solution (1x10⁶ cells/mL). RNase (20 µg/mL) solution was added followed by 1 mL of propydium iodide solution (PI, 50 µg/mL in PBS). Samples were incubated at 37 °C for 15 min, and PI fluorescence was analyzed using a EPIS XL analyzer. Compared to cisplatin (CDDP), platinum (IV)-polysaccharide conjugate at low concentrations (2.5 and 5 µg/mL) significantly enhanced the apoptotic effect on platinum-resistant ovarian cancer cells (Figures 6-7).

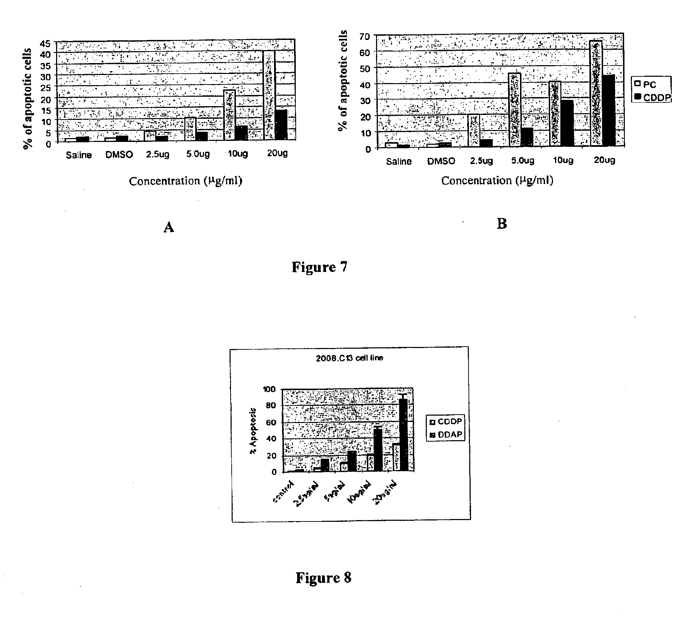

These results were confirmed by TUNEL assay, which, after 48 hours of treatment, shows a clear dose-dependent increase of apoptotic cells was detected after exposure to both drugs. However, when compared at each dose, platinum-polysaccharide conjugate (PC) treated group had many more cells experiencing apoptosis (P<0.05) (Figure 8).

### Example 3 - Evaluation of anticancer effect using breast tumor-bearing rat model:

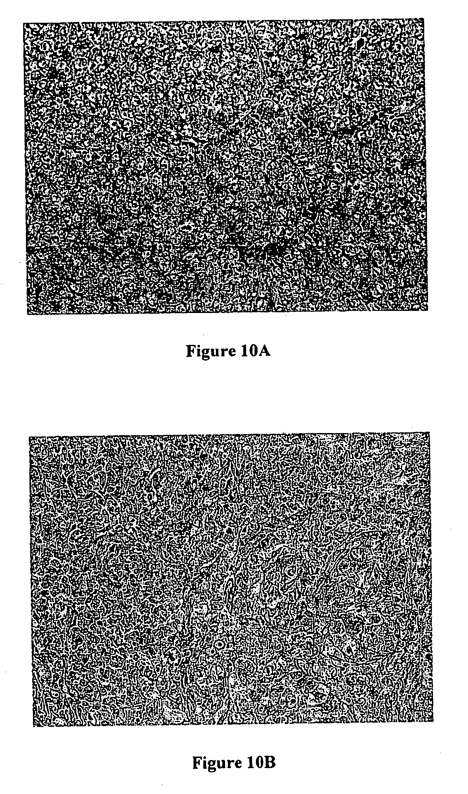

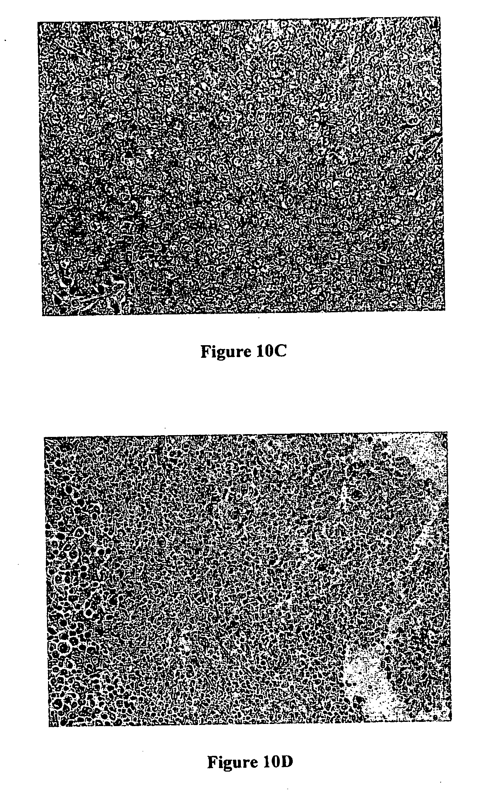

Female Fischer 344 rats (125-175g) were inoculated with breast cancer cells (13762NF, 10⁶ cells/rat, s.c. in the hind leg). After 15-20 days and a tumor volume of 1 cm, the breast tumor-bearing rats were administered either the platinum-chondroitin (Platinum-polysaccharide) conjugate (PC) or chondroitin alone at doses of 10 mg Pt/kg (platinum (IV)-polysaccharide) or 45 mg/kg (chondroitin). Tumor volumes and body weight were recorded daily for sixty days. Tumor volumes were measured as [length (1) x width (w) x thickness (h)]/2. Loss of body weight of 15% is considered a chemical-induced toxic effect. The results indicate that the platinum-polysaccharide conjugate (PC) is *effective in vivo* against breast tumor growth (Figure 9). After treatment with platinum-polysaccharide conjugate, tumor tissues were dissected and embedded in formalin. The tumor tissue was fixed in paraffin, and stained with hematoxylin and eosin for histological examinations. Extensive necrosis was observed at 94 hrs post-administration of platinum-polysaccharide conjugate, but not polysaccharide alone (Figure 10).

### Example 4 - Method of Tumor Cell Death or Inhibition:

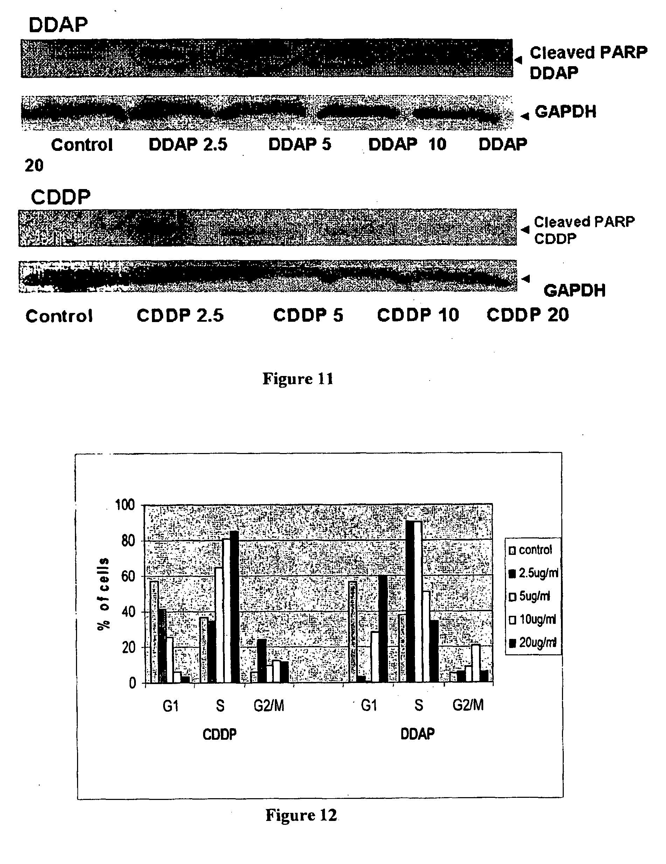

The effect of the platinum-polysaccharide conjugate of Example 1 on tumor cells was analyzed by treating 2008-c13 breast cancer cells with the conjugate, then analyzing the effects on cellular proteins through a Western blot (Figure 11). Cleaved PARP was significantly increased in the cells treated with platinum-polysaccharide conjugate (PC), compared with cisplatin (CDDP), suggesting that platinum-polysaccharide conjugate inhibited 2008-c13 cell growth through enhancement of apoptosis in a caspase 3 dependent pathway.

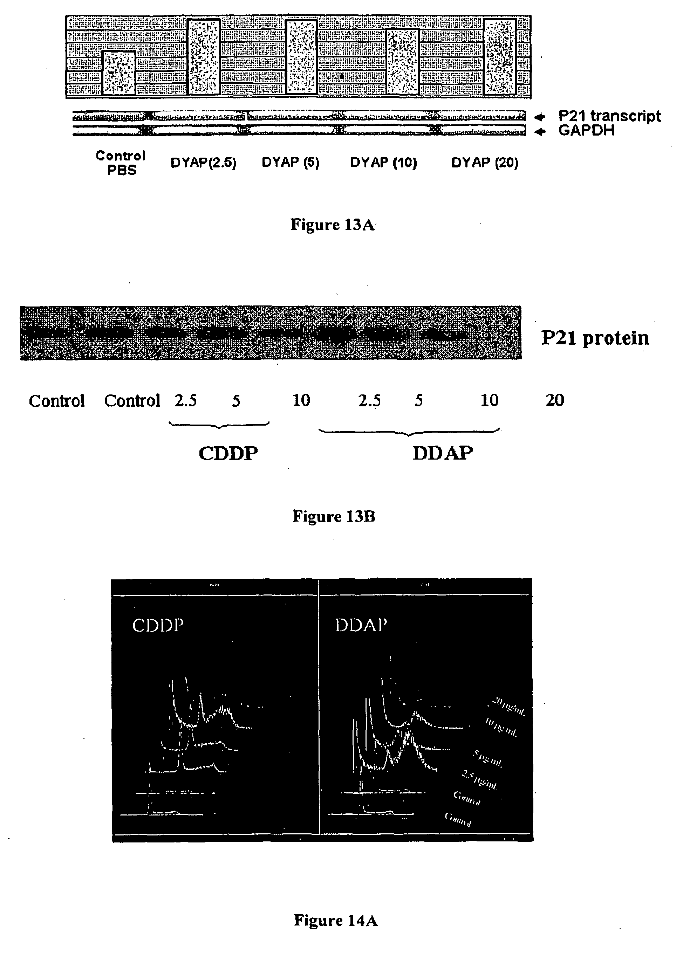

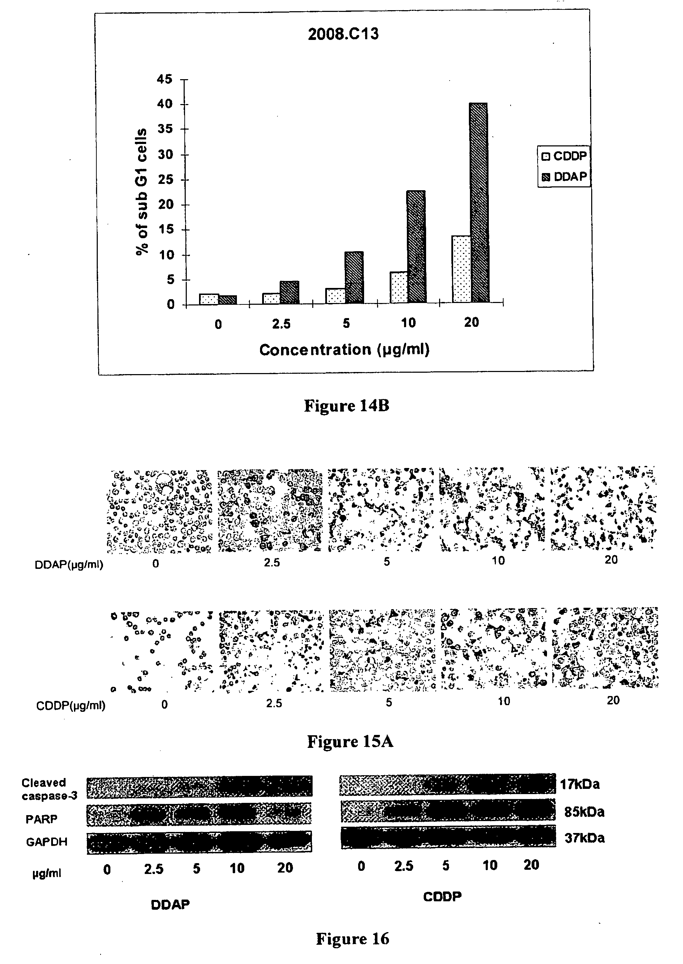

This effect was tested by flow cytometry in the 2008.C13 cell line after 48 hours and 72 hours of drug exposure. Flow cytometric analysis showed that there was a dose-dependent increase in the number of cells in the sub-G1 fraction after PC and CDDP treatments, which represents hypodiploid cells and indicates the induction of apoptosis. However, the use of PC, compared with CDDP, resulted in a more pronounced increase in the sub-G1 fraction at the same doses (Figure 14).

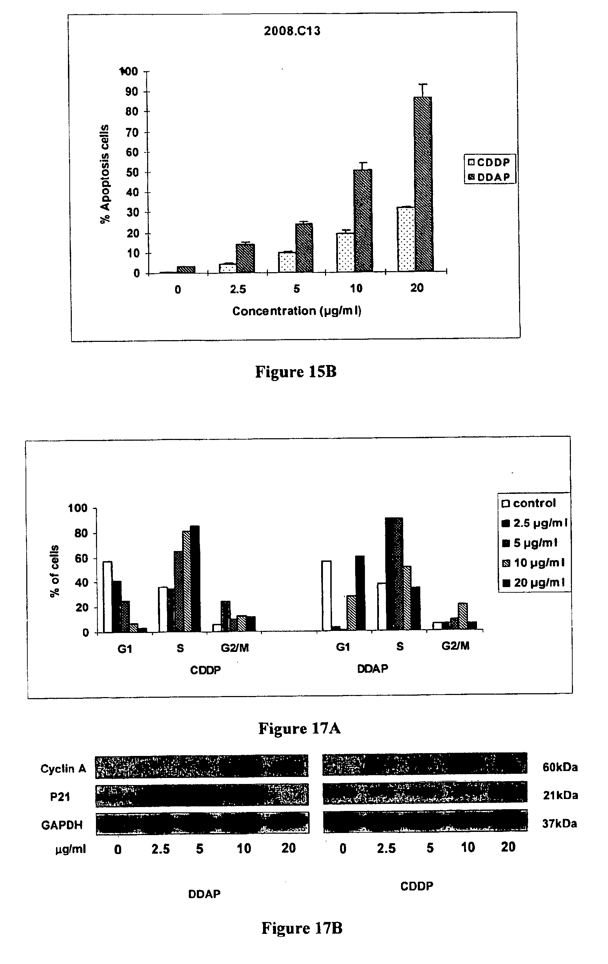

DNA fragmentation typical of apoptosis was further determined by the TUNEL assay in three independent experiments. A clear dose-dependent increase in the number of apoptotic cells was detected after exposure to both drugs. However, when compared at each dose, the PC-treated cells exhibited much higher levels of apoptosis (P < 0.05) (Figure 15).

To determine whether apoptosis is induced through a caspase-3-dependent pathway followed bv the cleavage of PARP, levels of cleaved caspase-3 and PARP, which form after caspase-3 activation, were determined by Western blot analysis. PARP is a 113-kDa nuclear protein that has been shown to be specifically cleaved to an 85-kDa fragment during caspase-3-dependent apoptosis. After cells were exposed to CDDP or PC for 48 hours, cleaved PARP was present at each dose. In the CDDP-treated group, cleaved PARP expression increased from 2.5 µg/mL to 20 µg/mL and cleaved caspase-3 was expressed in a pattern similar to that of PARP. In the PC-treated group, the expression of cleaved caspase-3 was comparable to that in the CDDP-treated group, except for the lower expression seen at 5 µg/mL of PC. Although cleaved PARP expression induced by high-dose (20 µg/mL) PC appeared to be lower than that induced by low-dose PC, no such difference was detected in its upstream cleaved caspase-3 expression (Figure 16).

In a further test on 2008-c13 breast cancer cells *in vitro,* flow cytometric analysis showed that cells significantly arrested in S-phase after exposure to platinum-polysaccharide conjugate (PC) at 48 hours (Figure 12). The highest levels of S-phase blockage happened at lower dosages of 2.5 and 5 ug/ml (90.3% and 90.1%). When compared with cisplatin (CDDP), the effect of platinum-polysaccharide conjugate (PC) on arresting cells in S-phase is significantly different.

Specifically, DNA content was analyzed by flow cytometry 48 hours after 2008.C13 cells were treated with PC or CDDP. Exposure to CDDP induced cell arrest in the S-phase and increased the sub-G1 fraction at the 5 µg/mL dose, but not at the lowest dose, 2.5 µg/mL. The numbers of cells arrested in the S phase and sub-G fraction increased continuously as the CDDP dose increased, with the maximal S-phase arrest (84.8%) occurring at 20 µg/mL. After cells were exposed to PC for 48 hours, the highest levels of S-phase block occurred at the lower doses (2.5 µg/mL [90.3%] and 5 µg/mL [90.1%]). At higher doses (10 and 20 µg/mL), the level of S-phase arrest steadily decreased as the sub-G1 fraction increased. This can be explained by the fact that under the strong stress of high-dose PC, cells underwent apoptosis promptly and directly before they were arrested in the S-phase (Figure 17).**

To elucidate the mechanism underlying S-phase arrest caused by CDDP and PC in 2008.C13 cells, the expression of p21 and cyclin A, which are important for cell-cycle regulation in the S phase, was examined in 2008.C13 cells after 48 hours of drug exposure. Neither p21 nor cyclin A expression was related to the extent of S-phase arrest after CDDP treatment. After PC treatment, however, p21 and cyclin A expression were directly related to the extent of S-phase arrest: p21 was up-regulated with maximal S-phase arrest after low-dose PC treatment, but not after high doses; cyclin A was up-regulated after high-dose PC treatment and was maintained at a low level after low-dose PC treatment (Figure 17B

2008-c13 breast cancer cells treated with platinum-polysaccharide conjugate (PC) showed increased p21 expression at both transcriptional (Figure 13A) and protein expression levels (Figure 13B) as compared to cells treated with cisplatin (CDDP).

## Claims

1. A polysaccharide conjugate comprising:
a chondroitin;
at least one aspartic acid; and
at least one molecule having platinum
wherein said polysaccharide conjugate is represented by the following formula:

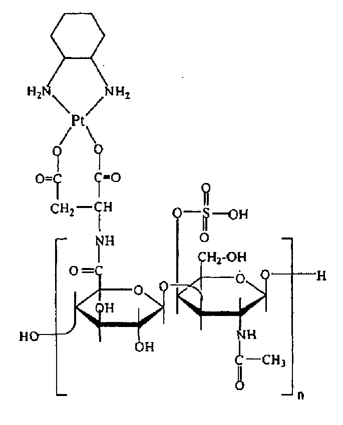

2. The polysaccharide conjugate of Claim 1, wherein the polysaccharide conjugate has a molecular weight of between about 20,000 daltons and about 50,000 daltons.

3. The polysaccharide conjugate of Claim 1, wherein the conjugate comprises between about 10% to about 50% by weight of said aspartic acid.

4. The polysaccharide conjugate of Claim 1, wherein the conjugate comprises between about 10% to about 50% by weight of said molecule having platinum.

5. The polysaccharide conjugate of Claim 1, wherein the conjugate comprises between about 15% to about 30% by weight of said molecule having platinum and approximately 70% by weight of said aspartic acid, and wherein the polysaccharide conjugate has a molecular weight of between about 26,000 daltons and about 30,000 daltons.

6. A method of synthesizing a polysaccharide conjugate comprising:
covalently bonding an aspartic acid to a chondroitin; and
conjugating a molecule having platinum to an O-group of said aspartic acid to form a polysaccharide conjugate;
wherein said polysaccharide conjugate is represented by the following formula:

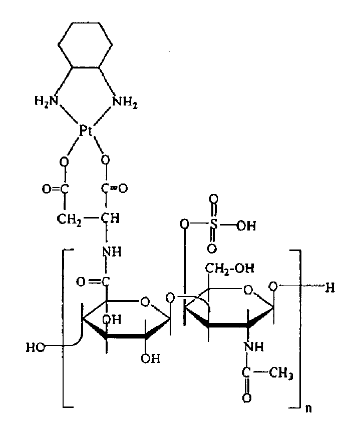

7. The method of Claim 15, further comprising drying the polysaccharide conjugate to form a powder.

## Patentansprüche

1. Polysaccharid-Konjugat, das ein Chondroitin, mindestens eine Asparaginsäure und mindestens ein Molekül mit Platin umfasst, wobei das Polysaccharid-Konjugat durch die folgende Formel dargestellt wird:

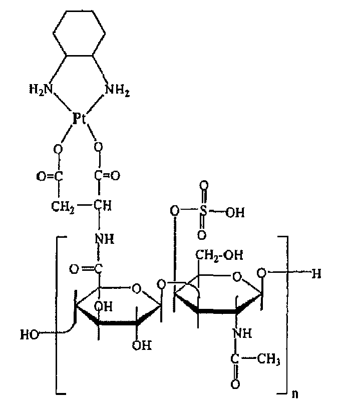

2. Polysaccharid-Konjugat gemäß Anspruch 1, bei dem das Polysaccharid-Konjugat ein Molekulargewicht zwischen etwa 20.000 Dalton und etwa 50.000 Dalton aufweist.

3. Polysaccharid-Konjugat gemäß Anspruch 1, bei dem das Konjugat zwischen etwa 10 und etwa 50 Gew.-% Asparaginsäure umfasst.

4. Polysaccharid-Konjugat gemäß Anspruch 1, bei dem das Konjugat zwischen etwa 10 und etwa 50 Gew.-% des Moleküls Platin umfasst.

5. Polysaccharid-Konjugat gemäß Anspruch 1, bei dem das Konjugat zwischen etwa 15 und etwa 30 Gew.-% des Moleküls Platin und etwa 70 Gew.-% Asparaginsäure umfasst und bei dem das Polysaccharid-Konjugat ein Molekulargewicht zwischen etwa 26.000 Dalton und etwa 30.000 Dalton aufweist.

6. Verfahren zur Synthese eines Polysaccharid-Konjugates, Schritte umfassend, bei denen man Asparaginsäure kovalent an Chondroitin bindet und ein Molekül mit Platin mit einer O-Gruppe der Asparaginsäure konjugiert, um ein Polysaccharid-Konjugat zu bilden, wobei das Polysaccharid-Konjugat durch die folgende Formel dargestellt wird:

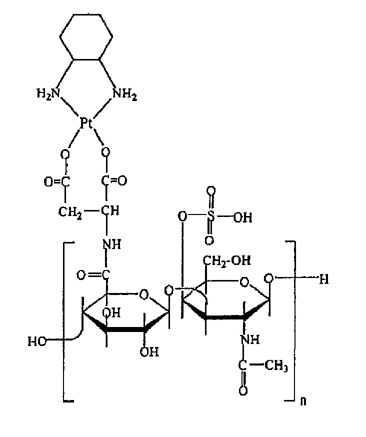

7. Verfahren gemäß Anspruch 15, bei dem man ferner das Polysaccharid-Konjugat trocknet, um ein Pulver zu bilden.

## Revendications

1. Conjugué de polysaccharide comprenant :
une chondroïtine ;
au moins un acide aspartique ; et
au moins une molécule contenant du platine ;
lequel conjugué de polysaccharide est représenté par la formule suivante :

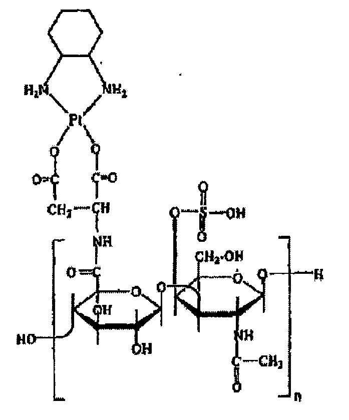

2. Conjugué de polysaccharide selon la revendication 1, lequel conjugué de polysaccharide a un poids moléculaire compris entre environ 20 000 daltons et environ 50 000 daltons.

3. Conjugué de polysaccharide selon la revendication 1, lequel conjugué comprend entre environ 10 % et environ 50 % en poids dudit acide aspartique.

4. Conjugué de polysaccharide selon la revendication 1, lequel conjugué comprend entre environ 10 % et environ 50 % en poids de ladite molécule contenant du platine.

5. Conjugué de polysaccharide selon la revendication 1, lequel conjugué comprend entre environ 15 % et environ 30 % en poids de ladite molécule contenant du platine et environ 70 % en poids dudit acide aspartique, et lequel conjugué de polysaccharide a un poids moléculaire compris entre environ 26 000 daltons et environ 30 000 daltons.

6. Procédé de synthèse d'un conjugué de polysaccharide comprenant :
la liaison covalente d'un acide aspartique à une chondroïtine ; et
la conjugaison d'une molécule contenant du platine à un groupe O dudit acide aspartique pour former un conjugué de polysaccharide ;
dans lequel ledit conjugué de polysaccharide est représenté par la formule suivante :

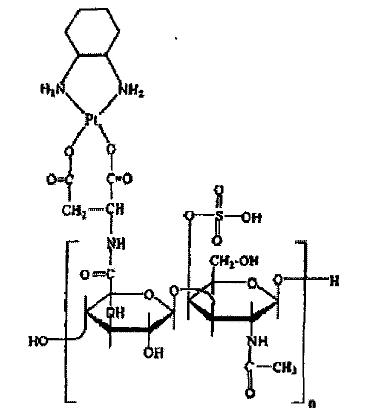

7. Procédé selon la revendication 15, comprenant en outre le séchage du conjugué de polysaccharide pour former une poudre.
